# EUROPEAN PATENT SPECIFICATION

(11) **EP 1 257 831 B1**
(45) Date of publication and mention of the grant of the patent: **03.05.2006**
(21) Application number: 01905964.1
(22) Date of filing: 21.02.2001
(51) Int. Cl.: G01N 33/82

(54) **DIAGNOSIS OF CVD MEASURING FOLATE AND HOLO-TCII**
DIAGNOSE VON HERZ- UND GEFÄSSKRANKHEITEN DURCH BESTIMMUNG VON FOLSÄURE UND HOLO-TCII
ANALYSE BIOLOGIQUE SERVANT A LA DETECTION D'UNE MALADIE CARDIOVASCULAIRE

(30) Priority: 21.02.2000 GB 0004040
(43) Date of publication of application: 20.11.2002
(73) Proprietor: AXIS-SHIELD ASA, 0510 Oslo (NO)
(72) Inventor: SUNDREHAGEN, Erling Axis-Shield ASA, N-0510 Oslo (NO); ORNING, Lars Axis-Shield ASA, N-0510 Oslo (NO)
(74) Representative: Cockbain, Julian
(86) International application number: PCT/GB2001/000747
(87) International publication number: WO 2001/063298

(56) References cited:
- US-A- 4 273 757
- US-A- 4 680 273
- FLYNN MARGARET A ET AL: "Atherogenesis and the homocysteine-folate-cobalamin triad: Do we need standardized analyses?" JOURNAL OF THE AMERICAN COLLEGE OF NUTRITION, vol. 16, no. 3, 1997, pages 258-267, XP001058251 ISSN: 0731-5724
- MATHEW L ET AL: "THE CLINICAL DIAGNOSIS OF MEGALOBLASTIC ANEMIAS IN INFANCY AND CHILDHOOD" INDIAN JOURNAL OF PEDIATRICS, vol. 51, no. 411, 1984, pages 429-442, XP001058208 ISSN: 0019-5456
- METZ J ET AL: "The significance of subnormal serum vitamin B-12 concentration in older people: A case control study." JOURNAL OF THE AMERICAN GERIATRICS SOCIETY, vol. 44, no. 11, 1996, pages 1355-1361, XP001058253 ISSN: 0002-8614
- WICKRAMASINGHE S N ET AL: "Limited value of serum holo-transcobalamin II measurements in the differential diagnosis of macrocytosis." JOURNAL OF CLINICAL PATHOLOGY (LONDON), vol. 49, no. 9, 1996, pages 755-758, XP001058213 ISSN: 0021-9746

## Description

The present invention relates to an assay method for detecting potential cardiovascular disease (CVD) in a vascularized subject, e.g. a human or non-human animal, especially a mammal, and in particular to an assay method which may be used to detect potential cardiovascular disease before the onset of CVD symptoms noticeable by the subject.

Cardiovascular disease is a major source of ill health among the human population yet early or preemptive treatment, e.g. with change of diet, reduction or cessation of smoking, increase in regular exercise, prescription of lipid lowering drugs, etc., has a high success rate.

Flynn et al. in J. Am. Coll. Nutrition **16** 258-267 (1997) studdied various parameters including folate and holo-TCII but did not conclude that holo-TCII was a suitable marker for CVD. Wickramasinghe et al. in J. Clin. Pathol. **49** 755-758 (1996) studied a number of parameters including holoTCII and folate in macrocytosis.

There is accordingly a need for methods which can be used to detect CVD or the potential for or propensity to CVD before the disease has progressed beyond the stage where treatment is routinely successful or may be used to prevent further progression of the disease, and in particular to detect CVD at the early stages when the symptoms are not apparent to the patient or his physician.

Such methods may be used to screen the general population, or at-risk groups within the population, e.g. males over 40, workers in high stress jobs, patients with unhealthy diets, smokers, etc. where potential CVD or propensity to CVD is diagnosed, preemptive treatment may be given and/or the patient may be encouraged to make adjustments to lifestyle and habits. Likewise, where CVD, potential CVD or propensity to CVD is detected, the patient may be submitted to further testing, e.g. using more expensive or time consuming techniques, such as ECG, with and without physical activity, radioisotope imaging of myocardial perfusion, X-ray (e.g. CT) myocardial angiography, MR myocardial angiography or perfusion imaging, etc. to confirm the presence and status of CVD. By the use of such assay methods as a "coarse filter" screening technique, unnecessary use of such expensive and time-consuming tests may be avoided while still increasing the likelihood of as-yet undiscovered CVD being found and treated before health damage becomes irreversible.

The present invention is based on the realization that the protein complex holo-transcobalamin II, (holo TCII) a complex of the carrier protein transcobalamin II (TCII) and Vitamin B₁₂ (cobalamin) and/or folate are efficient markers for cardiovascular disease, and in particular that abnormal low holo-TCII and/or folate levels in body fluids such as blood is indicative of CVD or susceptibility to CVD.

For the avoidance of doubt, the term "cobalamin" is used herein synonymously with "vitamin B₁₂" and includes all forms of vitamin B₁₂ (e.g.cyanocobalamin; 5-6-dimethyl-benzimidazolyl cyanocobamide; methylcobalamine; 5'-deoxyadenosylcobalamin) as may occur and be metabolically active (when appropriately presented) in the body.

Vitamin B₁₂ (cobolamin) is a water soluble vitamin which forms part of the vitamin B complex found in foods. The core molecule consists of a corrin ring of four pyrole units which surround the essential cobalt atom. Cobalamin is the only vitamin which cannot be synthesised by animals or plants and must be absorbed from food in the gut. It can however be stored in the liver. It is synthesised by micro-organisms, in particular by anaerobic bacteria and yeasts.

Cobalamin functions in vivo as a co-enzyme and cobalamin enzymes catalyse three types of reaction: (i) intra-molecular rearrangements; (ii) methylations; and (iii) reduction of ribonucleotides to deoxyribonucleotides in some micro-organisms. In mammals, only two enzymic reactions, namely (i) and (ii) above, are known to require cobalamin as a co-enzyme.

In the process of digestion, a salivary protein called haptocorrin (which is also referred to in the art as R-binder or transcobalamins I and III collectively) binds cobalamin in the upper gastrointestinal tract forming a complex which passes through the stomach. Pancreatic enzymes digest the cobalamin-haptocorrin complex in the ileum, liberating cobalamin which is then bound to a protein called intrinsic factor, which is secreted by the gastric mucosa, to form a further complex. The cobalamin-intrinsic factor complex binds to a specific receptor in the lining of the terminal ileum, whereupon it is dissociated by a releasing factor and the cobalamin is transported actively across the membrane of the ileum into the blood stream.

Cobalamin does not circulate in the body in a free form in any appreciable amount. Probably 99% or so of cobalamin is bound by one of the transcobalamin proteins (TC I, II and III) or albumin.

The protein believed to be solely responsible for transporting cobalamin to target tissues is transcobalamin II (TCII), a critical trace protein without which cobalamin cannot cross cell membranes. Despite this important metabolic function, only about 6-25% of cobalamin in the serum is bound to TCII - most is carried by haptocorrin. TCII comprises a single chain polypeptide of about 40 kDa found primarily in serum, seminal fluid and cerebro-spinal fluid. Cobalamin bound TCII (i.e. holo-TCII) attaches to specific receptors on cell membranes and, once bound, the holo-TCII is taken into cells by pinocytosis. The holo-TCII constitutes the metabolically active pool of cobalamin, since none of the other cobalamin binding proteins, including transcobalamins I and III, are able to facilitate entry of the vitamin into cells.

TCII is synthesised by the liver, vascular endothelium, enterocytes, macrophages and fibroblasts and circulates predominantly as apo-TCII, i.e. lacking bound cobalamin. It has a short half life of approximately 90 minutes.

Less than about a quarter of the total plasma cobalamin is associated with TCII. The rest is bound to the other transcobalamins or albumin as mentioned above. The function or role of the non-TCII transcobalamins is unclear, but since they bind both cobalamin and cobalamin-like substances, they may play a role in ensuring that potentially harmful analogues of cobalamin cannot compete with cobalamin by virtue of them being unable to enter cells if bound to TC I or III. They may play a role in removing cobalamin analogues from the circulation or may serve as a store of cobalamins. Alternatively, they may ensure that free cobalamin and analogues thereof are not available for utilisation by micro-organisms.

Folate (folic acid in its anionic form) is a vitamin belonging to the B vitamin complex, and is required as a cosubstrate in the formation of methionine from homocysteine. Its reduced form, tetrahydrofolate, is essential in the process of DNA biosynthesis.

Thus viewed from one aspect the invention provides an assay method for the detection of cardiovascular disease (CVD), potential cardiovascular disease, or propensity to cardiovascular disease, in a human or non-human animal subject, said method comprising assessing the concentration of holo-transcobolamin II (holo TCII) in a cobalamin containing sample from said subject, e.g. a sample of blood, plasma, serum, seminal fluid, amniotic fluid or cerebrospinal fluid, preferably a sample of blood, plasma or serum, in particular a sample of serum, and optionally further assessing the concentration of folate in the said sample.

By assessing it is meant that a quantitative or semi-quantitative value for the concentrations of holo-TCII and/or folate are determined. This may be a value for the concentration of the sample as tested, e.g. after treatment to remove cells or other sample components not being assayed for, or to concentrate or dilute the sample or to transfer the holo-TCII to a separate medium, e.g. a solid substrate.

Alternatively, the assessment may simply be qualitative, ie. to indicate whether the holo-TCII and/or folate concentrations are above or below one or more pre-selected threshold values, e.g. values indicative of absence of CVD detectable by the assay, presence of CVD (or potential CVD or propensity to CVD) as detectable by the assay, or uncertainty as to presence or absence of CVD, etc. The precise values for such threshold values or other reference values for holo-TCII and/or folate concentration may depend on the nature of the sample, the age, weight, sex and species of the subject and may be determined in a routine manner by testing equivalent subjects without CVD or with CVD at various stages of development.

A value indicative of holo-TCII concentration determined (or "assessed") in accordance with the method of the invention may be an absolute concentration of holo-TCII or may alternatively be an index, ratio, percentage or similar indication of the concentration of holo-TCII and that of some other analyte, e.g. another transcobolamin or homocysteine. A preferred ratio is that between the concentration of holo-TCII and the total cobolamin concentration. Total cobolamin assays are known from the literature as are assays for other analytes such as homocysteine which was mentioned above.

The body sample used in the assay method of the invention may be any cobalamin containing sample, e.g. a body fluid or tissue sample, or a suspension etc. Generally the sample will not be urine or a sample taken from the gastrointestinal tract. Preferably, the sample will be a body fluid for example, seminal fluid, cerebro-spinal fluid or amniotic fluid, or more particularly blood or a blood derived sample. When this is the case, the sample used for analysis will preferably be cell-free and hence either serum or plasma may be used. The sample may be treated prior to being used in the assay method of the invention, for example it may be diluted by adding a buffer or other aqueous medium.

While assays for holo-TCII are known and may be used in the method of the invention, there has not previously been any suggestion that holo-TCII is a marker for CVD or propensity to CVD.

Examples of holo-TCII assays are described or referenced for example in: Herzlich et al., Lab. Invest. 58: 332-337 (1988); Markle, Critical Reviews in Clinical Laboratory Sciences 33: 247-356 (1996); Herbert, Am. J. Clin. Nutrition 59 (5 Suppl.): 1213S-1222S (1994); Das et al., J. Nutr. Biochem. 2: 455-464 (1991); van Kapel et al., Clin. Chim. Acta 172: 297-310 (1988); Lindemans et al., Clin. Chim. Acta 132: 53-61 (1983); Nexø et al., Scand. J. Lab. Invest 37: 723-728 (1997); Morelli et al., J. Lab. Clin. Med. 89: 645-652 (1977); Carmel, Am. J. Clin. Pathol. 62: 367-372 (1974); Wickramasinghe et al., J. Clin. Pathol. 46: 537-539 (1993); Vu et al., Am. J. Hematol. 42: 202-211 (1993); Benhayoun et al., Acta Haematol. 89: 195-199 (1993); Rothenberg et al., Methods in Enzymology 281: 261-268 (1997); and in Frater-Schröder et al., pages 877-880 in "Vitamin B₁₂", Zagalak et al (Ed), W. De Gruyter, Berlin, 1979.

Thus for example van Kapel et al. (supra) disclose a method for specifically separating TCII from other transcobalamins using heparin sepharose, thus facilitating the quantitation of holo-TCII by radioisotope dilution assay and the concentration of non-cobalamin carrying TCII by measuring the unsaturated cobalamin binding capacity of the bound TCII with radioactive cobalamin. Similar methods using microfine silica such as QUSO™ have been used to bind TCII and allow its purification (in either apo or holo form) from TC I and III (see Das et al. (supra)). It is thought however that heparin sepharose is a more specific binder of TCII and some researchers have reported that TC I and III bind to silica in appreciable amounts (see Benhayoun *et al*. Acta Haematol. 89:195-199 (1993)). Toft *et al.* Scand. J. Clin. Lab. Invest. 54:62 (1994)) have recently proposed a method whereby transcobalamin II is adsorbed to cellulose and the cobalamin associated with the bound TCII may be quantified by standard methods.

An immunoassay for holo-TCII in which sepharose anti-TCII is used as described in Lindemans (supra) which uses a technique described by Lindemans et al. in Clin. Chim. Acta 95: 29-33 (1979).

The method currently used in clinical practice for determining holo-TCII involves adsorbing TCII to silica and then assaying the bound fraction for cobalamin content using either an immunoassay (as described for example by Kuemmerle et al. Clin. Chem. 38/10: 2073-2077 (1992) or a microbiological assay, the latter apparently producing the best results. This method is accurate and reliable.

In general, besides the sample under evaluation, calibration samples with known holo-TCII and/or known folate content will also be assessed in the performance of the assay method. Such determinations can be used to plot a calibration curve from which the holo-TCII and/or folate content of the sample under investigation may be determined. The nature of the calibration samples and selection of conversion or adjustment factors used in the determination of the holo-TCII and/or folate may vary depending, for example, on the manner in which holo-TCII or folate is detected in the assay technique actually used and on other aspects of the method which affect the assay result, for example, buffer composition, assay conditions etc. Typically, calibration samples having holo-TCII contents of 0 to 300 pmol/L will be used. The reference range within which the value for holo-TCII will generally be found is 0 to 160 pmol/L. A holo-TCII concentration in serum below 35 pmol/L will generally be strongly indicative of deficiency.

A set of cobalamin standards, preferably with an extended concentration range of 80 to 800 pmol/L or broader, e.g. 0 to 1500 pmol/L, may be used to determine the total cobalamin content of the sample, and not just the holo-TCII content, if such a measurement is required.

Typically, calibration standards having folate contents of 0-45 nM will be used. A folate concentration below 3.4 nM is considered low, as the range in a normal population is between 3.4 and 38 nM.

Besides obtaining a determination of holo-TCII and/or folate content for the sample under investigation, it may frequently be desirable to determine the total cobolamin content in the sample and/or the apo-TCII content in the sample. Many of the publications referred to above describe how this may be done.

Measurement of total cobalamin content of a sample, particularly a serum sample, may be desired to give an indication of any cobalamin imbalance over the period leading up to the time of sampling. Such measurement may be conducted in combination with assessment of holo-TCII levels and/or folate levels and forms a further aspect of the invention. Measurement of total cobalamin content in a sample is preferably carried out in combination with assessment of both holo-TCII levels and folate levels and may be achieved by any of the methods described in the publications referred to above.

In general, serum total cobolamin content for humans will be in the range 200-600 pmol/L and holo, TCII content will normally represent some 6 to 20% of this, ie. 30-160 pmol/L. A threshold value below which the assay may be held to be predictive of CVD or CVD propensity may generally be about 35 pmol/L, more preferably about 30 pmol/L especially about 20 pmol/L.

However, the threshold values are better calculated from holo-TCII determinations using the same assay technique for the same body sample type from a range of patients of similar type (age, sex, weight, species, etc.) from healthy through early stage CVD to serious CVD. Even more preferably, the threshold values will be values determined for the same patient at an earlier, healthy stage.

The present invention relates to a dual assay system measuring both total folate and holo-TCII in a sample, preferably a serum sample and preferably simultaneously or sequentially. The measurement of folate may be effected by first adding releasing or denaturing agent (such as one containing sodium hydroxide, potassium cyanide and dithiotreitol). A dual tracer containing cyanocobalamin radiolabelled Co57 and folate radiolabelled I125 can be added, followed by a limited amount of dual binder containing immobilized intrinsic factor and folate binder. The same dual tracers and binders may be used to quantify the level of holo-TCII. The concentration of TCII-bound cobalamin in a serum is determined from a standard curve constructed by using holo-TCII calibrators and the concentration of folate from a standard curve constructed of known amounts of folate.

Viewed from a further aspect, the present invention provides the use of an assay kit in a method according to any of claims 1 to 6, said kit comprising reagents as specified in claim 7 and optionally instructions for the performance of the assay method and for the interpretation of the results and, optionally, holo-TCII and/or folate containing reference samples, and optionally, a detector.

The instructions in the kit may for example be in the form of a label, a manual or an instruction leaflet; however they may instead take the form of a computer program or a data carrier, e.g. a computer disc.

The detector, where present, will generally be one capable of detecting a reporter species, e.g. a spectrometer, a nuclear radiation detector, a scattered light detector, etc.

The reagents will be reagents suitable for holo-TCII determination, e.g. reagents as specified in the literature cited herein which relates to holo-TCII determination.

The invention will now be described in the following non-limiting examples:

### EXAMPLE 1

### Clinical study on holo-TCII and cardiovascular disorders

Holo-TC II and homocysteine levels were measured in serum samples taken from (i) 25 healthy volunteers, (ii) 90 PTCA (Percutaneous Transluminal Coronary Angioplasty) patients prior to procedure, and (iii) 80 myocardial infarct patients six days after infarct and for 37 of these also six weeks after infarct.

Holo-TC II was measured using the non-specific method of adsorption of TC II on silica (Toft *et al*. (1994) Scand. J. Clin. Lab. Invest. 54: 62-63) and homocysteine by the IMx method developed by Axis (Shipchandler & Moore (1995) Clin. Chem. 41: 991-994).

35 pM was defined as the cut-off for holo-TC II; values below 35 pM being considered as deficient. For homocysteine 14.6 pM was defined as the cut-off; values below 14.6 pM were considered to be within the normal range.

As an estimate of risk, odds ratios were calculated as follows: cases with "out of normal" values/total cases of the disorder divided by the same ratio for the control group.

A value greater than one (1) indicates that a risk may exist.

| **Group** | **Total** | **Holo-TC II** | | **Homocysteine** | |
|---|---|---|---|---|---|
| | | Cases | Odds Ratio | Cases | Odds Ratio |
| Control | 25 | 1 | - | 1 | - |
| PTCA | 90 | 5 | 1.4 | 19 | 5.3 |
| MI, day 6 | 80 | 2 | 0.6 | 30 | 9.4 |
| MI, week 6 | 37 | 4 | 2.7 | 15 | 10 |

The odds ratio for homocysteine are in accordance with numerous other studies showing that homocysteine values higher than about 15 *µ*M are associated with a greater risk for cardiovascular disease.

The odds ratio for holo-TC II indicate that such a risk, albeit lower pertain also to holo-TC II. The values are probably underestimated because of the non-specific method used, adsorption of TC to silica. The oods ratio smaller than unity observed for MI at day 6 is most likely due to TC II being an acute phase protein and thus may be expected to increase in concentration after trauma such as a myocardial infarct. The increased concentration of TC II will cause a temporary redistribution of cobalamin from haptocorrin to TC II, masking any underlying chronic decrease in holo-TC II.

### Example 2

For measurement of holo-TCII, aliquots of serum are mixed for 30 min with an equal volume of PBS and magnetizable particles coated with antibodies specific for TCII. The magnetizable particles are sedimented by using a strong magnet and the supernatant removed. The particles are washed once and are subsequently treated with a releasing/denaturing reagent containing sodium hydroxide (0.3M), potassium cyanide (100 *µ*M), and dithiotreitol (15 mM). For measurement of folate, serum samples are directly treated with the releasing/ denaturing reagent. This will release substantially all bound cobalamin and folate, convert all cobalamin into the more stable cyanocobalamin form, and preserve the endogenous folate in reduced form. To all samples are then added a dual tracer containing cyanocobalamin radiolabeled with Co57 and folate radiolabeled with I125. A limited amount of a dual binder, containing immobilized Intrinsic Factor and folate binder, is added to each tube and incubated for 10-60 min. The binder is sedimented by centrifugation or by using a strong magnet, depending on mode of immobilization. The concentration of TCII-bound cobalamin in a serum sample is determined from a standard curve constructed by using holo-TCII calibrators and the concentration of folate from a standard curve constructed of known amounts of folate.

### Example 3

For measurement of holo-TCII, aliquots of serum are mixed for 30 min with an equal volume of PBS and magnetizable particles coated with antibodies specific for TCII. The magnetizable particles are sedimented by using a strong magnet and the supernatant removed. The particles are washed once and are subsequently treated with a releasing/denaturing reagent containing sodium hydroxide (0.3M), potassium cyanide (100 *µ*M), and dithiotreitol (15 mM). For measurement of total serum cobalamin and folate, serum samples are directly treated with the releasing/ denaturing reagent. This will release substantially all bound cobalamin and folate, convert all cobalamin into the more stable cyanocobalamin form, and preserve the endogenous folate in reduced form. To all samples are then added a dual tracer containing cyanocobalamin radiolabeled with Co57 and folate radiolabeled with I125. A limited amount of a dual binder, containing immobilized Intrinsic Factor and folate binder, is added to each tube and incubated for 10-60 min. The binder is sedimented by centrifugation or by using a strong magnet, depending on mode of immobilization. The concentration of TCII-bound cobalamin in a serum sample is determined from a standard curve constructed by using holo-TCII calibrators and the concentration of total serum cobalamin and folate from a standard curve constructed of known amounts of cobalamin and folate.

## Claims

1. An assay method for the detection of at least one disease from the set of cardiovascular disease and potential cardiovascular disease, in a human or non-human animal subject, said method comprising assessing the concentration of both holo-transcobolamin II (holo TCII) and folate in a cobalamin-containing sample from said subject.

2. A method as claimed in claim 1 wherein said sample is a sample of a fluid selected from the set of blood, plasma, serum, seminal fluid, amniotic fluid and cerebrospinal fluid.

3. A method as claimed in claim 1 or claim 2 wherein said sample is a blood sample.

4. A method as claimed in any one of claims 1 to 3 wherein said sample is a serum sample.

5. A method as claimed in any one of claims 1 to 4 wherein said sample is treated prior to use in the assay method to separate the holo-TCII.

6. A method as claimed in any one of claims 1 to 5 additionally comprising the measurement of the total cobalamin level in said sample.

7. Use of an assay kit in a method according to any one of claims 1 to 6, said kit comprising a denaturing agent, a dual tracer containing cyanocobalamin radiolabelled Co⁵⁷ and folate radiolabelled I¹²⁵, and a limited amount of dual binder containing immobilized intrinsic factor and folate binder.

8. Use as claimed in claim 7 wherein said kit additionally comprises holo-TCII containing reference samples.

9. Use as claimed in either of claims 7 and 8 wherein said kit additionally comprises folate containing reference samples.

## Revendications

1. Procédé d'analyse pour la détection d'au moins une maladie parmi l'ensemble constitué d'une maladie cardio-vasculaire et d'une maladie cardio-vasculaire potentielle, chez un sujet animal humain ou non humain, ledit procédé comportant l'évaluation de la concentration à la fois de holo-transcobalamine II (holo-TCII) et de folate dans un échantillon contenant de la cobalamine provenant dudit sujet.

2. Procédé selon la revendication 1, dans lequel ledit échantillon est un échantillon d'un fluide sélectionné parmi l'ensemble constitué de sang, de plasma, de sérum, de fluide séminal, de fluide amniotique et de fluide céphalo-rachidien.

3. Procédé selon la revendication 1 ou 2, dans lequel ledit échantillon est un échantillon sanguin.

4. Procédé selon l'une quelconque des revendications 1 à 3, dans lequel ledit échantillon est un échantillon de sérum.

5. Procédé selon l'une quelconque des revendications 1 à 4, dans lequel ledit échantillon est traité avant d'être utilisé dans le procédé d'analyse pour séparer la holo-TCII.

6. Procédé selon l'une quelconque des revendications 1 à 5, comportant de plus la mesure du niveau de cobalamine totale dans ledit échantillon.

7. Utilisation d'une trousse d'analyse dans un procédé selon l'une quelconque des revendications 1 à 6, ladite trousse comportant un agent dénaturant, un traceur double contenant une cyanocobalamine radiomarquée par Co⁵⁷ et du folate radiomarqué par I¹²⁵, et une quantité limitée d'un liant double contenant un facteur intrinsèque immobilisé et un liant de folate.

8. Utilisation selon la revendication 7, dans laquelle ladite trousse comporte de plus des échantillons de référence contenant une holo-TCII.

9. Utilisation selon la revendication 7 ou 8, dans laquelle ladite trousse comporte de plus des échantillons de référence contenant du folate.

## Patentansprüche

1. Prüfverfahren zum Erkennen zumindest einer Krankheit aus der Gruppe von kardiovaskulären Krankheiten und potentiellen kardiovaskulären Krankheiten in einem menschlichen oder nichtmenschlichen tierischen Subjekt, wobei das Verfahren das Überprüfen der Konzentration von sowohl Holo-Transcobalamin II (Holo TCII) als auch von Folat in einer Cobalamin enthaltenden Probe von dem Subjekt umfasst.

2. Verfahren nach Anspruch 1, bei dem die Probe eine Probe einer Flüssigkeit ist, die aus der Gruppe bestehend aus Blut, Plasma, Serum, Samenflüssigkeit, Fruchtwasser und Gehirn-Rückenmarksflüssigkeit ausgewählt ist.

3. Verfahren nach Anspruch 1 oder 2, bei dem die Probe eine Blutprobe ist.

4. Verfahren nach einem der Ansprüche 1 bis 3, bei dem die Probe eine Serumprobe ist.

5. Verfahren nach einem der Ansprüche 1 bis 4, bei dem die Probe vor der Verwendung in dem Prüfverfahren behandelt wird, um das Holo-TCII zu separieren.

6. Verfahren nach einem der Ansprüche 1 bis 5, das zusätzlich das Messen des gesamten Cobalaminniveaus in der Probe umfasst.

7. Verwendung einer Prüfausstattung in einem Verfahren nach einem der Ansprüche 1 bis 6, wobei die Ausstattung ein Denaturierungsmittel, einen Doppelindikator, der mit Co⁵⁷ radioaktiv markiertes Cyanocobalamin und mit I¹²⁵ radioaktiv markiertes Folat enthält und eine begrenzte Menge eines Doppelbindemittels, das einen immobilisierten Intrinsic-Factor und einen Folatbinder enthält.

8. Verwendung nach Anspruch 7, bei der die Ausstattung zusätzlich Holo-TCII enthaltende Referenzproben umfasst.

9. Verwendung nach Anspruch 7 oder 8, bei der die Ausstattung zusätzlich Folat enthaltende Referenzproben umfasst.
